# EUROPEAN PATENT APPLICATION

(11) **EP 3 482 712 A1**
(43) Date of publication of application: **15.05.2019**
(21) Application number: 17823357.3
(22) Date of filing: 05.07.2017
(51) Int. Cl.: A61C 3/02, A61B 17/16

(54) **DEVICE WITH MILLIMETRIC GRADING FOR OSTEOTOMY**

(30) Priority: 05.07.2016 US 201662358483 P
(71) Applicant: Brites, Débora, 22451-000 Rio de Janeiro (BR)
(72) Inventor: Brites, Débora, 22451-000 Rio de Janeiro (BR)
(74) Representative: Algemeen Octrooi- en Merkenbureau B.V.
(86) International application number: PCT/BR2017/050182
(87) International publication number: WO 2018/006151

(57) **Abstract**

The purpose of the present invention is to provide a device with millimetric grading for osteotomy and a method of treatment for bone tissue osteotomy using said device with millimetric grading in periodontal plastic surgery for aesthetic purposes and periodontal surgery to increase the clinical crown for prosthetic purposes.

## Description

### I - FIELD OF THE INVENTION

The present invention belongs to the field of bone tissue osteotomy in periodontal plastic surgery for aesthetic purposes and periodontal surgery to increase the clinical crown for prosthetic purposes.

Particularly, the present invention comprises a surgical drill, which features a rod with millimetric grading, and an active diamond tip substantially spherical, and can have varying diameters according to the surgery case and the amount of bone to be removed.

### II - BACKGROUND OF THE INVENTION

### 11.1 - Biological space

The integrity of the "biological space" is of the utmost importance to maintain healthy gums, as the existence thereof is crucial for adhesion of the junctional epithelium and connective tissue attachment to the tooth structure (Wagenberg *et al.,* 1989).

Maynard & Wilson conceptualized biological distances as being the dimension of the periodontium between the alveolar bone crest and the free gingival margin, characterized by the oral sulcular epithelium, the junctional epithelium and the connective tissue attachment (Maynard & Wilson, 1979). Gargiulo *et al.* carried out a work to verify the average biological distances at different eruptive stages of the teeth and obtained distances that did not change in these phases, with the following average measurements: Gingival Sulcus: 0.69 mm, Junctional Epithelium: 0.97 mm, and Connective Tissue Attachment: 1.07mm. These values mean it is possible to extrapolate as a general rule that there is a range of 2 to 3 mm of healthy tooth structure coronal to the alveolar bone crest to the junctional epithelium and connective tissue attachment to adequately join the dental element (Gargiulo *et al.*; 1961).

When the biological space of the periodontium is invaded, an inflammatory reaction in the periodontium typically occurs, which may induce alveolar bone resorption to provide space for new connective tissues, and to lead to a deepening of the gingival sulcus. This restoration of periodontal attachment in a more apical position and the deepened groove depth combined with marginal restoration often leads to a chronic inflammation and localized periodontal collapse (Allen, 1993).

### 11.2 - GINGIVAL PHENOTYPE

There is a correlation between the gingival thickness and the susceptibility to gum recession, when surgical or restoration procedures are performed. Therefore, making an accurate diagnosis of the gingival phenotype is of utmost importance when preparing a treatment plan designed to achieve an esthetically favorable and predictable result (Kan *et al.,* 2010).

The characteristics of the gingival thickness, the height of the attached gum, the underlying alveolar bone thickness and the shape of the dental crowns have been used to classify gingival phenotypes (Egreja *et al.,* 2012; Stellini *et al.,* 2013). Seibert & Lindhe, classified the gums into two phenotypes: thick and thin (Seibert & Lindhe, 1989).

The thick gum phenotype is characterized by the existence of dense fibrous tissue, a broad band of keratinized gum and a lack of festooning between the interdental papillae and gingival vestibular. The thin gum phenotype is friable, having a high risk of recession after surgical/repair therapy (Ahmad, 2005). The thin gum tissue has a more transparent appearance, being more delicate and highly sensitive to trauma and inflammation and is associated to a thin vestibular alveolar bone (Esfahrood *et al.,* 2013).

The shape and position of each tooth in the arch are also capable to influence the dimensions of the biological space. As in the case of restoration, the biological space must also be respected when lengthening the crown, and it is important to pay attention to two aspects: the amount of bone that has to be removed in order to maintain the minimum dimensions required to maintain healthy gums, and the gingival phenotype of the patient (Schmidt *et al.,* 2013).

### 11.3 - INCREASE OF CLINICAL CROWN FOR PROSTHETIC PURPOSES AND ESTHETIC PERIODONTAL PLASTIC SURGERY

The surgery to increase the clinical crown in the periodontal field can have two purposes: to provide a healthy periodontium in restorative procedures and to correct a gummy smile (esthetic periodontal plastic surgery).

### II.3.1 - INCREASE OF CLINICAL CROWN FOR PROSTHETIC PURPOSES

In many clinical situations, the ideal conditions for carrying out the restorative procedure are not present, the professional having to seek alternatives to create access to the cervical wall of the cavity in order to enable an operating field free from contamination and moisture. In situations where this occurs, only surgical-periodontal procedures can promote favorable conditions (Cueva, 2000). One of the options of surgical-periodontal procedures that can bring benefits to the restorative treatment is the surgery for clinical crown increase.

When the biological space is invaded, the organism promotes the reabsorption of supporting bone tissue to compensate the lost space, resulting in the formation of a periodontal pocket and alveolar bone loss. Consequently, it is recommended to maintain a space of at least 3 mm between the gingival margin and the alveolar bone crest (Carranza & Newman, 1997). Accordingly, for successful restorative treatment without damaging the supporting tissue, the surgery to the increase of clinical crown is indicated. It is common to find subgingival cavity margins invading the space consistent with the biological space (corresponding to the sulcus epithelium, junctional epithelium and connective tissue attachment), thus requiring surgical intervention to restore the conditions of normality to the supporting tissues. In this sense, this surgery has been widely performed by promoting an increase in the size of the clinical crown, thus allowing improved restorative treatments.

Surgical procedures to increase clinical crown comprise the excision or soft tissue through gingivectomies and gingivoplasty or requiring the removal of bone tissue through osteotomies and osteoplasty. This type of surgery is primarily indicated when there is invasion of biological space, as this is of great importance when success is sought, or during restorative treatment.

Clinical crown increase procedures are performed in order to allow adequate preparation, either for the tooth to receive direct restorative treatment or for indirect molding and restoration. They are also indicated to adjust gingival margins in cases where improved esthetics are required (Newman *et al.,* 2004).

### II.3.2 - PERIODONTAL PLASTIC SURGERY FOR AESTHETIC PURPOSES

The current surgical procedures for the treatment of gummy smiles for aesthetic purposes have used various nomenclatures such as periodontal plastic surgery for aesthetic purpose, aesthetic crown increase (Cairo *et al.,* 2012; Malkinson *et al.,* 2013).

Periodontal plastic surgeries have been highly valued and is increasingly sought after by people who argue they want to fix the "gummy smile", which occurs when smiling, showing more gums than teeth.

Increasingly, patients' complaints relate to the esthetics of their smiles, making periodontal surgery for correcting gummy smiles more common in the daily life of the dental surgeon (Silva, 2008). The disharmonious smile due to excess gums compromises facial esthetics. The treatment plan is made after discovering the etiology and identifying the aesthetic results foreseen by the patient (Oliveira & Venturim, 2012).

One of the indications for the treatment of gummy smile is when the patient presents altered passive eruption. In these cases, the patient has normal jaw growth and lip positioning, however the gums are exposed and the crowns are short (Cairo *et al.*; 2012).

The distance between the bone crest and the cemento-enamel junction, ranging from 1.5 mm to 2 mm, is a decisive indication of bone remodeling, where there is no such distance, osteotomy is performed so that there is enough space to accommodate the connective tissue attachment, junctional epithelium and gingival sulcus (biological space) (Joly *et al.*; 2010). If this measure is not appropriate for every surgical case, gum recession may occur or excess gum may remain whereby hindering the expected aesthetic result.

Several treatments have been suggested for gummy smile correction in cases of altered passive eruption, including minimally invasive when not using flaps and bone exposure and those using the full flap for visualization of bone tissue facilitating the osteotomy (Ribeiro *et al.,* 2013).

### II.4 - DESCRIPTION OF THE SURGICAL TECHNIQUE

Many techniques have been proposed for the periodontal surgical treatment. Gingivectomy was defined in 1979 as an excision of soft tissue of a pathological periodontal pocket (Grant *et al.* 1979). Widman published the technique which was called "Original Widman Flap" where the flap technique was described to eliminate the periodontal pocket and bone recontouring to establish a new physiology for the alveolar bone (Widman; 1918). Neuman later proposed changes to the original technique by introducing intrasulcular incision and access to better root debridement (Neuman; 1920). Again in 1982, Neuman published an article showing the removal of the gingival collar after mucoperiosteal flap followed by osteotomy (bone leveling with a spherical drill) (Neuman; 1982).

After performing periodontal probing, which involves measuring the gingival margin to the alveolar bone crest, this measurement is transferred to the outer face of the gums, thus obtaining the markings with bleeding points in the gingival margin, the incision line thus being defined. The primary incision should be performed with a 15c scalpel blade, in internal or inverted bevel, for determining the amount of gum to be removed; the secondary is intrassulcular towards the alveolar crest and aims to highlight the gum collar previously incised and the third incision is interdental, made alongside the occlusal plane and, when necessary, relaxing incisions are indicated.

In cases where osteotomy is recommended, the full-thickness flap is debrided, with the aid of a delicate detacher (Molt), taking care to avoid tearing of the interdental papillae, and the internal bevel enables the appropriate amount of keratinized gingiva to be maintained. The osteotomy is performed when there is invasion of the biological space. This being the case, it must be carried out with the aid of chisels or spherical drills, with due regard for irrigation with syringe and abundant saline solution. The drill must be placed parallel to the long axis of the tooth and perpendicular to the alveolar bone crest. With the aid of a periodontal probe with millimetric grading, the distance of the bone crest is verified at the end of the preparation and, in aesthetic cases, JCE. When this is less than 3 mm, more osteotomy is needed.

If necessary, cervical osteoplasty will also be performed in the vestibular and palatal or lingual osteoplasty to regularize the bone tissue. Osteoplasty is indicated to give the bone tissue a better outline, restoring the bone anatomy and, consequently, promoting better settlement of the flap to the bone tissue and, consequently, returning a more harmonic and physiological contour to the patient's esthetics. The suture should be performed in order to preserve the papillae and to promote adequate coaptation of the lips.

By means of the osteotomy, the supporting bone tissue is worn in apical level so that it is possible to restore a physiological contour, as well as to return the biological distances consistent with the normality, in order to restore the health of the periodontal support tissues (Lindhe *et al.,* 2005).

In view of the above, it is crucial to develop devices that optimize the surgical procedure to increase the clinical crown for prosthetic and aesthetic purposes, so as to facilitate the osteotomy.

### III - BRIEF DESCRIPTION OF THE DRAWINGS

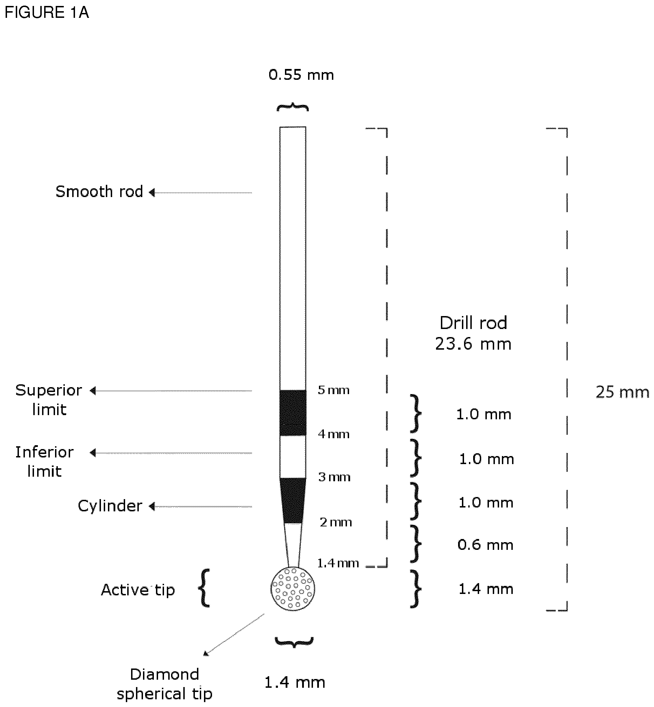

Figure 1A is a front view of the device with millimetric grading for osteotomy of the present invention.

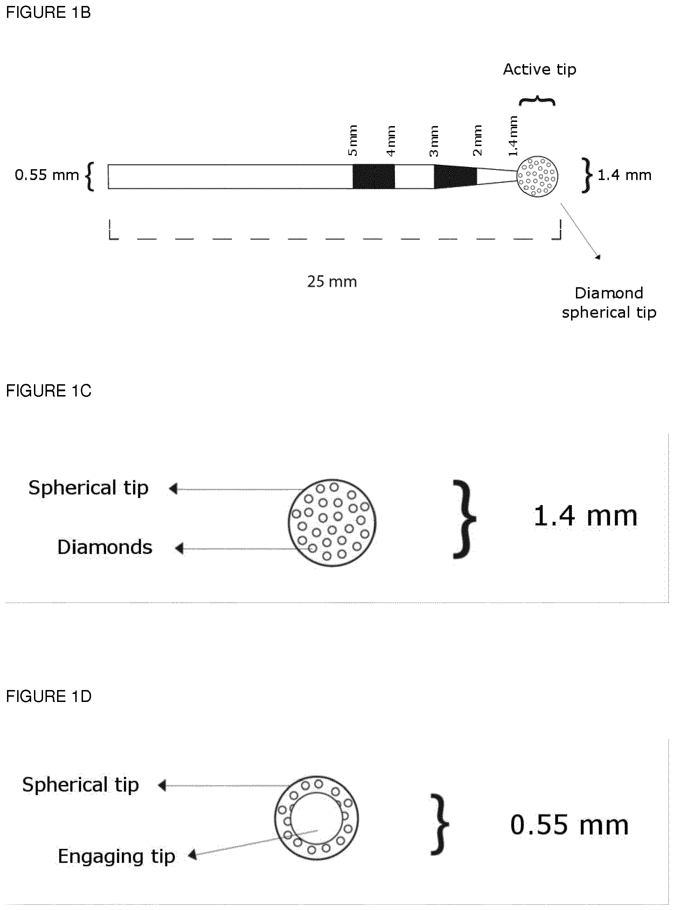

Figure 1B is a side view of the device with millimetric grading for osteotomy of the present invention.
Figure 1C is a bottom peripheral view of the device with millimetric grading for osteotomy of the present invention.
Figure 1D is a top peripheral view of the device with millimetric grading for osteotomy of the present invention, wherein it is possible to see both the tip of the rod which engages into the rotation handpiece, and the diamond active tip (spherical tip).

### IV - DETAILED DESCRIPTION OF THE INVENTION

Osteotomy consists of bone wear, directed from the end of the prosthetic preparation and JCE (cement-enamel junction) at the margin of the alveolar bone crest, which must have appropriate measure to provide the formation of a certain biological space during the process of wound healing and periodontium tissue maturation. Thus resulting in the formation of a healthy and aesthetic periodontium.

The measure of bone wear is related to the periodontal phenotype. The thicker the periodontium, the greater will be the measure of the end of the prosthetic preparation and JCE to the margin of the alveolar bone crest. A thick-plane periodontium requires maintaining a biological space of 4 mm, in a thick festooned periodontium, 3 mm, and in a fine-festooned periodontium, 2 mm.

In one aspect of the present invention, it is provided an elongated device with millimetric grading (drill) for bone tissue osteotomy, comprising a body consisting of stainless steel material (elongated rod), where the millimetric markings are defined, and a substantially spherical active tip, wherein the active tip is made of a material selected from a group consisting of uniform diamond material (natural and synthetic diamonds micrograins) or carbon-enriched tungsten carbide (carbide) and is located at one end of the drill, wherein the device has an elongated rod usually conical and cylindrical and the diameter of both the elongated rod and the active tip are much smaller than the length of the elongated rod.

After use, the drill (rod + active tip) can be sterilized in an autoclave and reused several times. During the surgical procedure, it is inserted into a high-rotation handpiece, that will operate long enough for the required osteotomy at a high speed and simultaneous irrigation of saline solution.

The drill rod presents a regular marking in order to enable visualization and interpretation of the millimeterage of the penetration depth reached by the drill during the surgical procedure. The marking can be made by various techniques which allow the visualization and interpretation of the millimeterage, not limiting the invention. The marking technique can be selected from the group consisting of marking by chemical ablation, mechanical ablation or laser ablation. The marking of the first point on the drill rod of the invention will vary according to the diameter of the active tip, so that the first marking is in a milmeter-whole value, measured from the end of the active tip, for example in 2.0 mm or 3.0 mm. The subsequent markings will be equidistant from each other. Preferably, the subsequent markings of the points in the rods are every 1.0 mm.

The active diamond tip has thick to average granulation, ranging from around 90 µm to 150 µm.

In a preferred aspect of the present invention, the rod of the device presents a substantially conical shaped at the part closest to the edge of the active tip and substantially cylindrical shape at the farthest part from the active tip, wherein the diameter of the active tip may vary from about 1.0 to 2.0 mm and the length of the elongated rod may range from about 22.0 to 25.0 mm, according to the surgical case and the amount of bone to be removed. The diameter of the elongated rod may vary from about 0.40 to 0.70 mm.

Even more preferably, the rod of the device of the present invention presents a cylindrical shape, at the farthest part from the active tip, with a diameter of about 0.55 mm and a conical shape at the closest part from the active tip, which is spherical and diamond, with a diameter of about 1.4 mm, and the elongated rod presents a length of about 23.6 mm, wherein the average speed of rotation of the device inserted into the rotation handpiece is around 350,000 rpm.

In another aspect of the present invention it is provided a treatment method for bone tissue osteotomy through the use of the device with millimetric grading of the present invention in periodontal plastic surgery for aesthetic purposes and periodontal surgery to increase the clinical crown for prosthetic purposes.

### V - EXAMPLE

The present invention comprises a drill that measures 25 mm, presents a rod with a 23.6 mm length and diameter of 0.55 mm, and a spherical diamond active tip, with a diameter of 1.4 mm. The rod has a conical shape (from the active tip up to the marking of 3.0 mm on the elongated device) and a cylindrical shape as of the marking of 3.0 mm marking.

The drill rod has millimetric grading (up to 5 mm) by means of marking by laser ablation, see FIGURES 1A and 1B, allowing the dental surgeon to wear the appropriate measure of bone tissue while operating, thus discarding the need to use the periodontal probe with millimetric grading during the entire osteotomy procedure. The first marking by laser ablation of the elongated rod is positioned at a distance of 2.0 mm measured from the bottom edge of the active tip. Subsequent markings are equidistant from each other, separated every 1.0 mm.

The use of the periodontal spherical drill with millimetric grading of the present invention optimizes the surgical procedure to increase the restorative and esthetic clinical crown, because it eliminates the need of measuring the desired space of the prosthetic margin and the JCE at the end of the bone crest by using the periodontal probe with millimetric grading. In other words, during osteotomy, the very drill of the present invention simultaneously performs this measure, eliminating the need to use a periodontal probe with millimetric grading.

## Claims

1. An elongated device with millimetric grading (drill) for bone tissue osteotomy **characterized in that** it comprises a body (elongated rod), where millimeter markings are defined, and an active tip located at one end of the drill, wherein the diameter of the rod and of the active tip is much smaller than the length of the elongated rod.

2. The device according to claim 1, **characterized in that** the active tip is made of a material selected from a group consisting of diamond material or carbon-enriched tungsten carbide (carbide).

3. The device according to claim 2, **characterized in that** the active tip is preferably made of a diamond material.

4. The device according to any one of claims 1 to 3, **characterized in that** the active tip presents a diameter ranging from 1.0 to 2.0 mm.

5. The device according to claim 4, **characterized in that** the active tip presents a diameter of 1.4 mm.

6. The device according to any one of claims 1 to 5, **characterized in that** the active diamond tip presents thick to medium granulation, ranging from 90 µm to 150 µm.

7. The device according to any one of claims 1 to 6 **characterized in that** the rod of the device presents a substantially conical shape in the closest part to the active tip and a substantially cylindrical shape in the farthest part from the active tip.

8. The device according to any one of claims 1 to 7, **characterized in that** the rod of the drill is made of stainless steel material and presents a diameter ranging from 0.40 to 0.70 mm and a length ranging from 22.0 to 25.0 mm.

9. The device according to claim 8, **characterized in that** the rod presents a diameter of 0.55 mm and a length of 23.6 mm.

10. The device according to any one of claims 1 to 9, **characterized by** being inserted during the osteotomy surgical procedure into a high-rotation handpiece that operates long enough for the osteotomy required at a high speed and concomitant saline solution irrigation made of a diamond material.

11. The device according to claim 10 **characterized in that** the average speed of rotation of the device inserted into the rotary handpiece is around 350,000 rpm.

12. The device according to any one of claims 1 to 11, **characterized in that** said millimetered markings are made by a technique chosen from the group consisting of chemical ablation, mechanical ablation and laser ablation, so that the markings enable visualization and interpretation of the millimeterage of the device during the surgical procedure.

13. The device according to claim 12, **characterized in that** said millimetered markings are made by a laser ablation technique.

14. A method of treatment for bone tissue osteotomy **characterized by** using the device with millimetric grading as defined in any one of claims 1 to 13 in periodontal plastic surgery for aesthetic purposes and periodontal surgery for increasing the clinical crown for prosthetic purposes.
